# EUROPEAN PATENT SPECIFICATION

(11) **EP 4 284 493 B1**
(45) Date of publication and mention of the grant of the patent: **04.12.2024**
(21) Application number: 22715385.5
(22) Date of filing: 30.03.2022
(51) Int. Cl.: A61N 1/36, A61N 1/40, A61N 1/04

(54) **CARRIER FOR ELEMENTS OF TUMOR-TREATING FIELD PROVISION SYSTEM AND SYSTEMS AND METHODS OF USING SAME**
TRÄGER FÜR ELEMENTE EINES TUMORBEHANDLUNGSFELDBEREITSTELLUNGSSYSTEMS SOWIE SYSTEME UND VERFAHREN ZUR VERWENDUNG DAVON
SUPPORT POUR ÉLÉMENTS DE SYSTÈME DE FOURNITURE DE CHAMP DE TRAITEMENT DE TUMEUR ET SYSTÈMES ET PROCÉDÉS D'UTILISATION DE CELUI-CI

(30) Priority: 31.03.2021 US 202163168416 P
(43) Date of publication of application: 06.12.2023
(73) Proprietor: Novocure GmbH, 6039 Root D4 (CH)
(72) Inventor: LEONARD II, Francis X., Malvern, PA 19355 (US)
(74) Representative: Boden, Keith McMurray
(86) International application number: PCT/IB2022/052951
(87) International publication number: WO 2022/208390

(56) References cited:
- US-A1- 2019 255 344
- US-B2- 8 447 395

## Description

### FIELD

This disclosure relates to systems and methods for providing Tumor-Treating Fields, and, in particular, to systems and methods for improving mobility of subjects receiving Tumor-Treating Fields.

### BACKGROUND

Tumor-Treating Fields (TTFs) can be used to treat various types of cancer. For example, TTFs can be applied to a portion of a body of a subject via one or more transducer arrays. Typically, the transducer arrays couple to a signal generator that generates the TTFs through the transducer arrays. The signal generator is typically carried in a handbag that resembles luggage, and one or more cables run between the transducer arrays and the signal generator.

The subject is, therefore, tethered to the signal generator by cables. If the subject inadvertently moves too far from the signal generator, the signal generator can be decoupled from the transducer arrays, thereby activating an alarm and drawing unwanted attention to the subject, or the transducer arrays can be pulled from a desired position on the body of the subject. Further, movement between the subject and the signal generator and the cables can cause strain on the cables (e.g., at joints or coupling locations of the cables) that can lead to general wear or breaking of the cables. Thus, a system that enables the subject to freely move around (e.g., stand, sit, walk, and exercise) without causing significant wear of or strain on the cables or requiring the subject to lift and carry the signal generator is desirable.

Still further, the handbag that resembles luggage is meant to be seen and noticed in order to minimize others passing by from tripping on or inadvertently knocking over the handbag. Accordingly, the handbag can draw unwanted attention and can be aesthetically unpleasing. A more discrete system is desirable.

US-A-8447395 discloses an apparatus for selectively destroying or inhibiting the growth of bacteria located within a target region of a patient including a generator which can be disposed within a carrying bag or the like (e.g., a bag that extends around the patient's waist) which is worn by the patient or can be strapped to an extremity or around the torso of the patient.

US-A-2019/0255344 discloses a magnetic field system which is configured to provide a magnetic field in a first direction to tissue of a subject and includes at least one magnetic field source to produce the magnetic field.

### SUMMARY

Described herein, in various aspects, is a system for applying Tumor-Treating Fields to a portion of a body of a subject according to claim 1.

Embodiments of the invention are disclosed in die dependent claims.

Additional advantages of the invention will be set forth in part in the description that follows, and in part will be obvious from the description, or may be learned by practice of the invention. The advantages of die invention will be realized and attained by means of the elements and combinations particularly pointed out in die appended claims. It is to be understood that both the foregoing general description and the following detailed description are exemplary and explanatory only and are not restrictive of the invention, as claimed.

### DESCRIPTION OF THE DRAWINGS

These and other features of the preferred embodiments of the invention will become more apparent in the detailed description in which reference is made to the appended drawings wherein:
FIG. 1 is a schematic diagram of a subject wearing a system as disclosed herein.
FIG. 2 is a front view of a subject in a standing position wearing a system as disclosed herein.
FIG. 3 is a partial top view of a subject in a seated position wearing the system as in FIG. 2.
FIG. 4 is a front view of an exemplary carrier of a system as disclosed herein.
FIG. 5A is a perspective view of an exemplary electrical connection box.
FIG. 5B is a schematic side view of another exemplary electrical connection box
FIG. 6 is a schematic diagram of a subject wearing various carriers at various locations as disclosed herein.
FIG. 7 is a block diagram of an operating environment comprising a computing system as disclosed herein.
FIG. 8A shows a rear view of a subject wearing a carrier in accordance with embodiments disclosed herein. FIG. 8B shows a side view of the subject and the carrier as in FIG. 8A.
FIG. 9A shows a front view of a subject wearing a carrier in accordance with embodiments disclosed herein. FIG. 9B shows a side view of the subject and the carrier as in FIG. 9A.
FIG. 10A shows a front view of a lower body of a subject wearing a carrier in accordance with embodiments disclosed herein. FIG. 10B shows a side view of the lower body of the subject and the earner as in FIG. 10A.

### DETAILED DESCRIPTION

The present invention now will be described more fully hereinafter with reference to the accompanying drawings, in which some, but not all embodiments of the invention are shown. Indeed, this invention may be embodied in many different forms and should not be construed as limited to the embodiments set forth herein; rather, these embodiments are provided so that this disclosure will satisfy applicable legal requirements. Like numbers refer to like elements throughout. It is to be understood that this invention is not limited to the particular methodology and protocols described, as such may vary. It is also to be understood that the terminology used herein is for the purpose of describing particular embodiments only, and is not intended to limit the scope of the present invention.

Many modifications and other embodiments of the invention set forth herein will come to mind to one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

As used herein the singular forms "a," "an," and "the" include plural referents unless the context clearly dictates otherwise. For example, use of the term "a transducer array" or "a strap" can refer to one or more of such transducer arrays or straps, and so forth.

All technical and scientific terms used herein have the same meaning as commonly understood to one of ordinary skill in the art to which this invention belongs unless clearly indicated otherwise.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. Optionally, in some aspects, when values are approximated by use of the antecedent "about," it is contemplated that values within up to 15%, up to 10%, up to 5%, or up to 1% (above or below) of the particularly stated value can be included within the scope of those aspects. Similarly, in some optional aspects, when values are approximated by use of the terms "substantially" or "generally," it is contemplated that values within up to 15 %, up to 10%, up to 5%, or up to 1% (above or below) of the particular value can be included within the scope of those aspects. When used with respect to an identified property or circumstance, "substantially" or "generally" can refer to a degree of deviation that is sufficiently small so as to not measurably detract from the identified property or circumstance, and the exact degree of deviation allowable may in some cases depend on the specific context.

As used herein, the terms "optional" or "optionally" mean that the subsequently described event or circumstance may or may not occur, and that the description includes instances where said event or circumstance occurs and instances where it does not.

As used herein, the term "at least one of" is intended to be synonymous with "one or more of." For example, "at least one of A, B and C" explicitly includes only A, only B, only C, and combinations of each.

The word "or" as used herein means any one member of a particular list and also includes any combination of members of that list.

It is to be understood that unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is in no way intended that an order be inferred, in any respect. This holds for any possible non-express basis for interpretation, including: matters of logic with respect to arrangement of steps or operational flow; plain meaning derived from grammatical organization or punctuation; and the number or type of aspects described in the specification.

The following description supplies specific details in order to provide a thorough understanding. Nevertheless, the skilled artisan would understand that the apparatus, system, and associated methods of using the apparatus can be implemented and used without employing these specific details. Indeed, the apparatus, system, and associated methods can be placed into practice by modifying the illustrated apparatus, system, and associated methods and can be used in conjunction with any other apparatus and techniques conventionally used in the industry.

Tumor Treating Fields (TTF) therapy is a proven approach for treating tumors using alternating electric fields at frequencies between 50 KHz - 1 MHz. The alternating electric fields can be induced by transducer arrays (e.g., arrays of capacitively coupled electrodes) placed on opposite sides of the subject's body. When an AC voltage is applied between opposing transducer arrays, an AC current is coupled through the transducer arrays and into the subject's body. In some embodiments, the frequency of the alternating voltage can be between 50 kHz and 1 MHz, or between 100 kHz and 500 kHz.

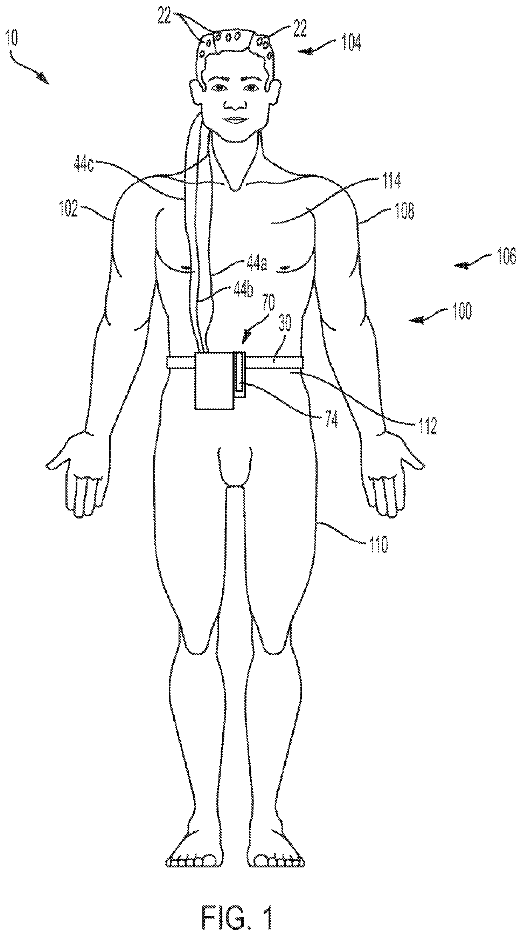

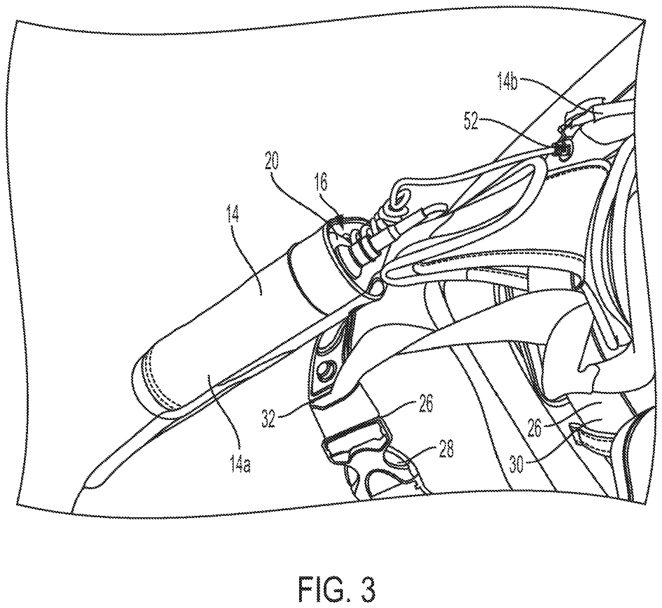

Disclosed herein, in various aspects and with reference to FIGS. 1-6 is a system for applying TTFs to a portion of a body 102 of a subject 100. As described herein, the body 102 of the subject 100 can include any portion of the subject, including, for example, a head 104, a torso 106, or limbs (e.g., upper arms 108, thighs 110) of the subject. The system 10 can comprise a carrier 12 having a carrier body 14 that defines a receiving space 16.

An electric-signal generator 20 can be at least partially received within the receiving space 16 of the carrier body 14. Optionally, the electric-signal generator 20 can be entirely received within the receiving space 16 of die carrier body 14. In further aspects, the electric-signal generator 20 can be only partially received within the receiving space 16 of the carrier body 14. The receiving space 16 can be configured to snugly receive the electric-signal generator 20 to inhibit movement thereof. For example, the electric-signal generator 20 can be elongate (e.g., optionally, generally cylindrical and elongate along the height of the cylinder), and the receiving space 16 can be elongate relative to a longitudinal axis to receive the electric-signal generator 20. In further optional aspects, the receiving space 16 can have therein a retaining strap that secures (optionally, fixedly secures) the electric-signal generator 20 within the receiving space.

The carrier 12 can further comprise a coupling apparatus 26 that is configured to fixedly couple the earner body 14 to a first location on the body 102 of the subject 100. In various aspects, the first location can be a thigh 110, a portion of the torso 106 (e.g., a waist 112 or a chest 114 or an annpit 116), or an upper arm 108 of the body 102 of the subject 100. In some optional aspects, the coupling apparatus can be configured to snugly retain the carrier body 14 against the body of the subject. Thus, in some optional aspects, the carrier body 14 cannot move (e.g., can move by no more than 5 cm in any direction) relative to the body of the subject without the subject intentionally moving the carrier body. In this way, the position of the carrier body 14 can stay substantially fixed on the body during daily activities. This is in contrast to conventional carriers that are meant to be carried by hand or worn loosely on the body and set down or removed during normal activities such as sitting in a chair or otherwise transitioning between seated and standing positions.

In various aspects, the coupling apparatus 26 can comprise a belt 30, a strap 32, and/or a sleeve 34. In some optional aspects, the coupling apparatus 26 can comprise a clip, buckle, hook and loop, or other fastener 28. The clip, buckle, hook and loop material, or other fastener 28 can be configured to enable the coupling apparatus 26 to be selectively secured in a looped configuration or in an open configuration. In further optional aspects, the coupling apparatus 26 can comprise a strap adjuster that is configured to adjust an operative length of the coupling apparatus (e.g., the circumference of the belt, strap, or sleeve). Optionally, the clip, buckle, hook and loop material, or other fastener 28 can serve as the strap adjuster.

It is contemplated that the coupling apparatus 26 can optionally be elastic (e.g., to allow elongation). In further aspects, the coupling apparatus 26 can be inelastic.

In one aspect, the coupling apparatus 26 can comprise a belt 30 that is configured to extend around the torso 106 of the subject. For example, the belt 30 can be configured to extend around the waist 112 of the subject (e.g., below the ribs of the subject and at or above the hips of the subject). Thus, in some aspects, the carrier 12 can be positioned at or above the waist 112 of the subject 100. For example, in some optional aspects, the carrier body 14 can be positioned at the lower back, at one of the hips, or in front of the pelvis of the subject. In further aspects, the belt 30 can extend around the chest 114 of the subject (e.g., around or across the ribs of the subject), In use, it is contemplated that the carrier 12 can be positioned sufficiently high enough (relative to the waist) to ensure that the carrier (and the device) do not contact the legs of the subject when the subject transitions from a standing position to a seated position.

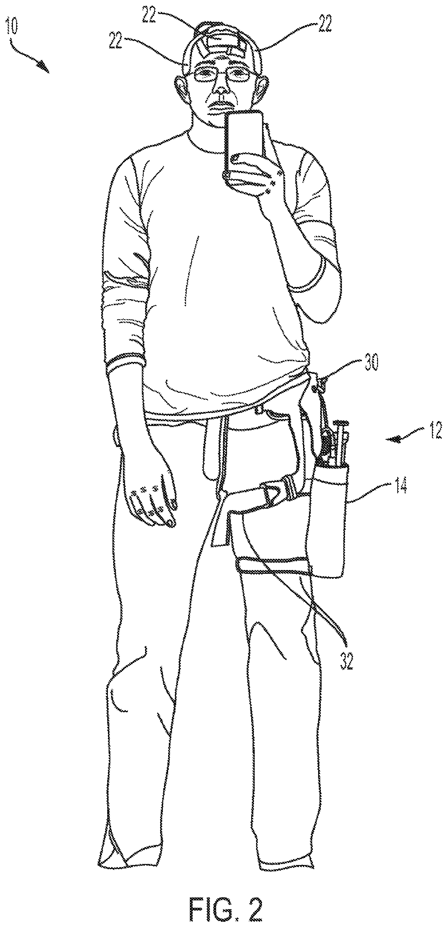

Referring to FIGS. 2-3, in further aspects, the coupling apparatus 26 can comprise a strap 32 that is configured to extend around a thigh 110 of the subject. Thus, the carrier body 14 can optionally be positioned on the side or front of the thigh 110.

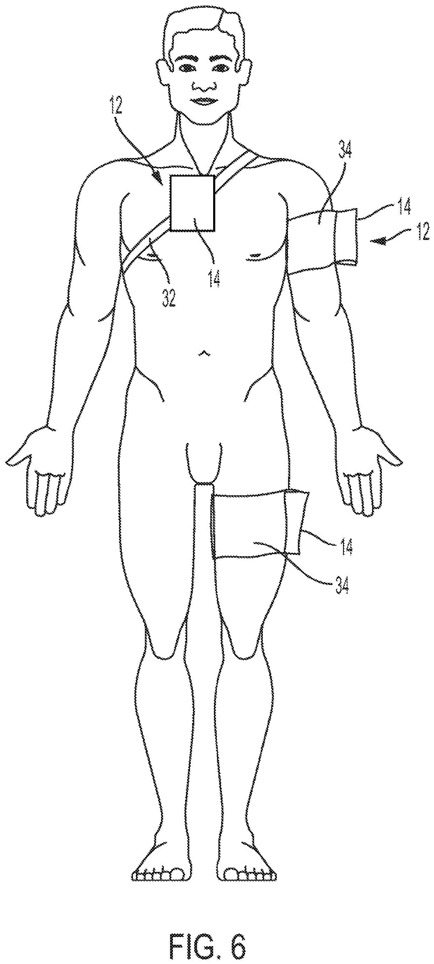

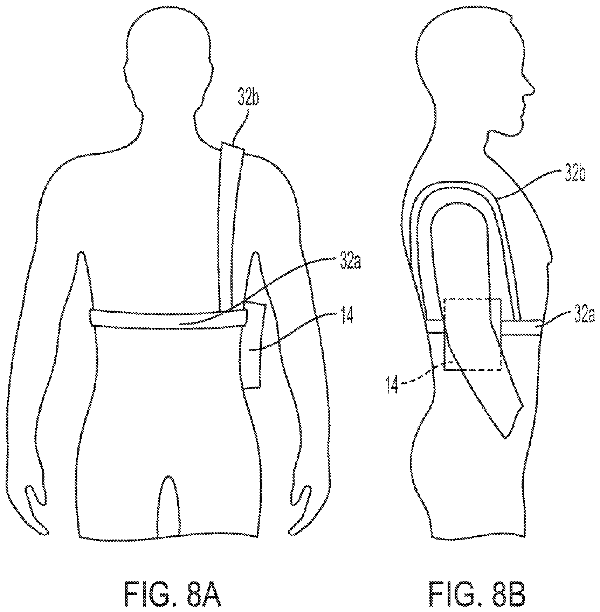

Referring to FIG. 6, in further aspects, the coupling apparatus 26 can comprise a strap 32 that is configured to extend over a shoulder of the subject and underneath an armpit on a side of the subject opposite the shoulder in order to position the carrier body 14 at the chest or within the armpit (and above the waist) of the subject. Referring also to FIGS. 8A-8B, the carrier body 14 can be positioned underneath and/or at least partially within the armpit of the subject. For example, in some aspects, the coupling apparatus 26 can comprise a torso strap 32a that extends around the torso (above the waist) of the subject and retains the carrier body 14 against the torso of the subject. The coupling apparatus 26 can further comprise at least one shoulder strap 32b that extends over at least one shoulder of the subject and distributes at least a portion of the weight of the carrier body 14 to the shoulder. In some optional aspects, the shoulder strap(s) 32b can couple to the torso strap 32a at first and second attachment points to the front and rear, respectively, of the carrier body 14. In further aspects, the shoulder strap 32b can couple to the carrier body 14.

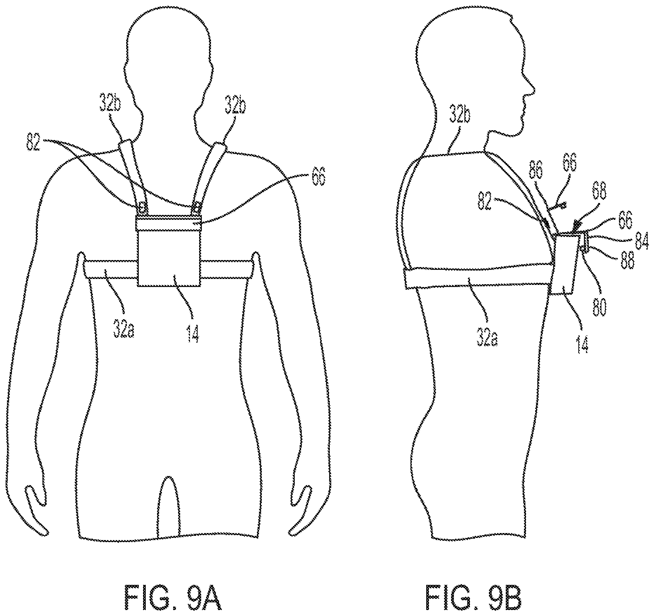

Referring to FIGS. 9A-9B, in some optional aspects, the carrier body 14 can be positioned on the chest of the subject. For example, the coupling apparatus 26 can comprise a torso strap 32a that extends around the torso (above tire waist) of the subject and retains the carrier body 14 against the torso of the subject. The coupling apparatus 26 can further comprise a first shoulder strap 32b that extends over a first shoulder of the subject and distributes at least a portion of the weight of the carrier body 14 to the first shoulder. The coupling apparatus 26 can further comprise a second shoulder strap 32b that extends over a second shoulder of the subject and distributes at least a portion of the weight of the carrier body 14 to the second shoulder. Respective first ends of the first and second shoulder straps 32b can couple to the torso strap 32a, and respective second ends of the first and second shoulder straps can optionally couple to either the carrier body 14 or the torso strap 32a.

Optionally, in use, the carrier body 14 can be configured to be held entirely above the lowest rib of the subject when worn by the subject. Optionally, in use, the carrier body can be configured to be held entirely below the neck of the subject when worn by the subject.

Referring to FIG. 6, in further aspects, the coupling apparatus 26 can comprise a sleeve 34. Optionally, the sleeve 34 can be configured to extend around the thigh 110 of the subject. In further aspects, the sleeve 34 can be configured to extend around the upper arm 108 of the subject.

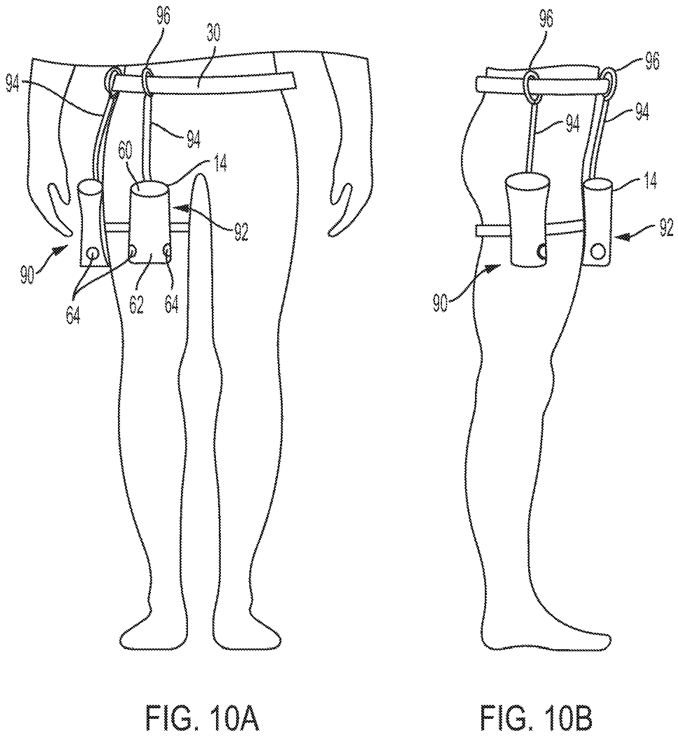

In some aspects, and as shown in FIG. 2, the carrier 12 can comprise a belt 30 that is configured to extend around the waist of the body of the subject. Optionally, the carrier housing 14 can hang from the belt 30 via a suspension strap 94 (FIG. 10A). Optionally, the length of the suspension strap 94 can be adjustable. The carrier can further comprise a strap 32 that is configured to extend around the thigh of the body of the subject. For example, at least one strap 32 (e.g., an upper strap and a lower strap, as shown) can couple the carrier body to the thigh. In this way, it is contemplated that the majority of the weight of the carrier can be supported by the waist of the subject, and the strap around the thigh can retain the carrier body against the thigh.

It is further contemplated that the belt 30, strap 32, or sleeve 34 can surround a body part (e.g., the leg, torso, or arm of the subject), and the carrier body 14 can be shifted about said body part (without decoupling the coupling apparatus from the subject) in order to adjust the position of the carrier body for different circumstances and environments. For example, as described, the carrier 12 can be worn about the waist of the subject. In some circumstances (e.g., when walking), it can be advantageous for the carrier body 14 to be in a first position (e.g., positioned at the lower back of the subject), and, in further circumstances (e.g., when sitting), the carrier body can advantageously be in a second position (e.g., at a hip or in front of the pelvis of the subject). Thus, the carrier 12 can enable the subject to reposition the carrier body 14 between the first position and the second position. Similarly, in various aspects, the carrier body 14 can be positioned on the front, side, rear, or inside of the thigh of the subject. For example, referring to FIGS. 10A-10B, in some aspects, the carrier body 14 can be worn on in a first position 90 on an outside of the thigh of the subject. In some aspects, this position (on the outside of the thigh to avoid contact with the other thigh) can be advantageous for walking, etc. In some aspects, the carrier body 14 can be shifted from the first position 90 to a second position 92 that is on a front of the thigh of the subject. In some aspects, it is contemplated that this position (on the front of the thigh) can be advantageous for sitting in a chair with armrests, which could potentially contact the carrier body 14 in the first position on the outside of the thigh. Optionally, to shift the carrier body 14 about and between the first and second positions 90, 92, both the carrier body 14 and the coupling apparatus 26 (e.g., the belt 30 and the strap 32) can shift together (e.g., rotate together relative to the subject). In further aspects, the carrier body 14 can shift (e.g., slide) relative to at least a portion of the coupling apparatus 26. For example, in some aspects, the suspension strap 94 can couple to the belt 30 via a loop 96 so that the loop is slidable along the length of the belt. Optionally, the carrier body can be movable along the length of the strap 32 around the thigh. In further aspects, the carrier body and strap 32 around the thigh can shift concurrently.

In various optional aspects, the carrier body 14 can be positioned at one of the chest, armpit, the upper arm, or the thigh. For example, optionally, the carrier body 14 can be positioned on the chest so that the carrier body is entirely above the rib cage and entirely below the neck (see FIGS. 9A-9B, for example). In further aspects, the carrier body 14 can be positioned on the chest so that the carrier body is positioned above the thighs of the body of the subject when the subject is seated. Optionally, the carrier body can be positioned at the armpit sufficiently low so that the carrier body does not interfere with arm movement (see FIGS. 8A-8B, for example). For example, the carrier body 14, when worn, can be configured to be positioned below the pectoralis major of the subject.

One or more transducer arrays 22 can be positioned on the body 102 of the subject 100. For example, the one or more transducer arrays 22 can be positioned on or within the head 104 of the subject. In further aspects, the transducer arrays can be positioned on the torso 106 of the subject 100 (e.g., for treating lung cancer) or other portions of the body of the subject. The one or more transducer arrays 22 can be in communication with the electric-signal generator 20 via at least one electrical cable 24. Each of the transducer arrays 22 can comprise one or more transducers that are configured to emit TTFs in response to signals provided by the electric-signal generator 20. The one or more transducer arrays 22 can be positioned on the body 102 of the subject 100 at a second location that is different from the first location.

The second location can be proximate to a treatment site. For example, and without limitation, the second location can be on the head 104 of the subject 100. In exemplary aspects, the second location can be a top of the head, a front of the head, a rear of the head, a side of the head, or an area that includes portions thereof. For example, a transducer array can cover at least a portion of a top, front, rear, and sides of the head of the subject as illustrated in FIG. 1. In further aspects, the second location can be on the torso of the subject. For example, the second location can be on a front, a back, or a side of the torso. In still further aspects, the transducer arrays 22 can be position anywhere on the body of the subject.

In further aspects, each transducer array 22 can be at a respective second location, wherein each respective second location is different from the first location. For example, die respective second locations on the body can be opposed sides of the head (e.g., left and right sides or front and back sides of the head). In further aspects, the respective second locations on the body can be on the torso (e.g., on front and rear sides of the torso). In still further aspects, the second location of at least one transducer array 22 can be on the torso of the body of the subject, and the second location of at least one additional transducer array 20 can be on a head of the body of the subject.

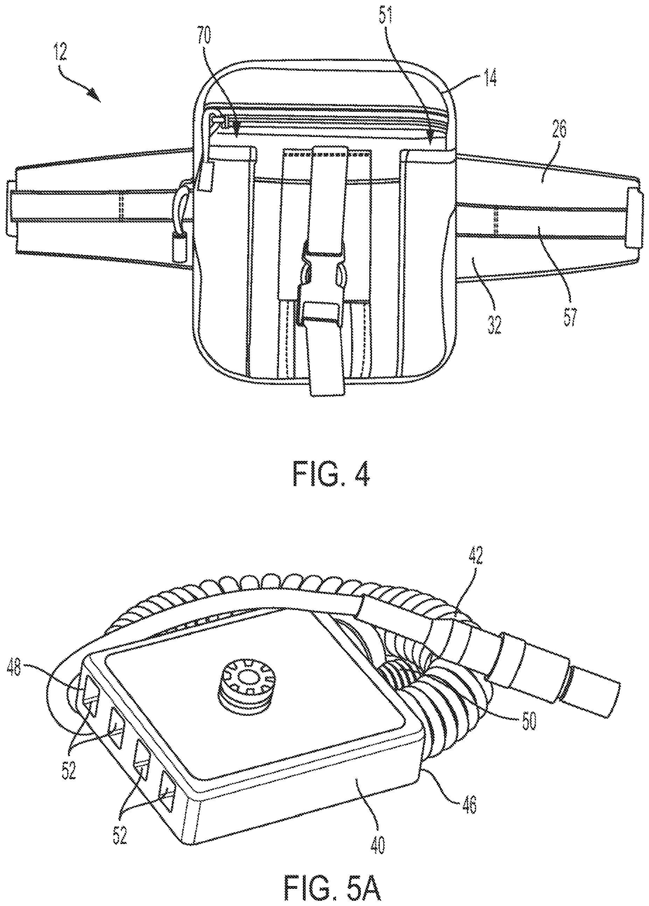

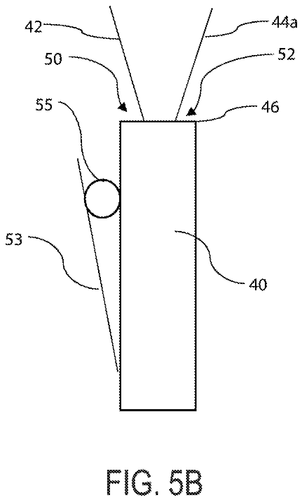

Optionally, in some aspects and with reference to FIGS. 1 and 5A, the system 10 can comprise an electrical connection box 40. The electrical connection box 40 can provide electrical communication (and a physical coupling) between the one or more transducer arrays 22 and the electric-signal generator 20. For example, in some aspects, the at least one electrical cable 24 can comprise a first electrical cable 42 that extends between and couples to the electric-signal generator 20 and the electrical connection box. A respective second electrical cable (e.g., electrical cables 44a, 44b, 44c) can extend between and couple to each transducer array 22 (e.g., transducer array 22a, 22b, 22c). In this way, the electrical connection box 40 can serve as a junction box that can enable the electric-signal generator 20 to deliver different electric signals to the different transducer arrays 22.

Referring to FIGS. 1 and 5A-5B, in some aspects, the electrical connection box 40 can have a first side 46 and an opposing second side 48. The first side 46 can define a first port 50 that couples to the first electrical cable 42, and the second side 48 can define at least one second port 52 that couples to the second electrical cable (e.g., the electrical cables 44a, 44b, 44c). In some aspects, at least one electrical cable can be integral to the electrical connection box 40. For example, the first electrical cable 42 can be integral or permanently physically coupled to the electrical connection box 40 through the first port 50. In further aspects, both the first port 50 and the second port(s) 52 can be on the same first side 46 of the electrical connection box. In this way, all of the electrical cables can extend from the electrical connection box in the same direction so that the electrical connection box 40 can be inserted into a receiving space with all the electrical cables exiting out a single opening of the carrier body 14 without significant bends (e.g., 90 degrees or more) in any of the electrical cables. In yet further aspects, the electrical connection box 40 can be omitted, and the electrical cable(s) 24 can extend directly from the electric-signal generator 20 to the transducer array(s) 22.

In some optional aspects, the electrical connection box 40 can couple to the carrier 12. For example, the system 10 can comprise a clip 53 that couples the electrical connection box to the carrier 12. In some optional aspects, the clip 53 can couple the electrical connection box 40 to the belt 30 or other coupling apparatus 26. Optionally, the clip 53 can comprise a member that is rotationally biased against a surface (e.g., of the electrical connection box 40) by a torsion spring 55. In some aspects, die coupling apparatus 26 can define a loop 57 (FIG. 4) that can receive the clip 53.

In various further aspects, the electrical connection box 40 can be received within the carrier 12. For example, optionally, the electrical connection box 40 can be received within the receiving space 16. In further optional aspects, the receiving space 16 can be a first receiving space, and the carrier 12 can define a second receiving space 51. In some aspects, the carrier 14 can comprise at least a first carrier body 14a and a second carrier body 14b, wherein the first carrier body defines the first receiving space 16, and the second carrier body defines the second receiving space 51. In further aspects, the carrier body 14 can define both the first and second receiving spaces 16, 5.1. Optionally, the electrical connection box 40 can be received within the second receiving space 51. In further aspects, the carrier can define at least one pocket 70 (FIG. 4). Optionally, the pocket(s) 70 can be separate from the receiving space(s).

Referring to FIGS. 10A-10B, in some aspects, the carrier body 14 can have an inner surface 60 and an outer surface 62. The inner surface 60 of the carrier body 14 can define the receiving space 16. In some optional aspects, the carrier body 14 can further define at least one opening 64 that extends between the inner surface 60 and the outer surface 62. The opening(s) 64 can be in communication with the receiving space of the carrier body and can be configured to permit dissipation of heat from the electric-signal generator 20. In some aspects, the carrier body can define a single opening 64. In further aspects, the carrier body can define 2, 3, 4, or more openings 64. In various optional aspects, the opening(s) 64 can be, for example, on the side(s) of the carrier body 14 and/or on the bottom of the carrier body. Optionally, a penneable cover, such as a grate, screen, or perforated material, can cover the one or more openings 64 while permitting air to pass therethrough.

Referring to FIGS. 9A-9B, in further aspects, the carrier 12 can comprise a cover 66 that couples to the carrier body 14 and extends over at least a portion of the receiving space 16 of the carrier body. The cover 66 can inhibit liquid or other undesirable elements (e.g., food crumbs, dust, etc.) from entering the receiving space. For example, in some aspects, the carrier body can define a top opening 68 to permit the electric-signal generator to be inserted into and removed from the carrier, and the cover 66 can extend across the top opening when in a closed position 84. Optionally, the cover 66 can pivotably couple to the carrier body 14 (e.g., via a hinge or flexible coupling). In further aspects, the cover can couple to the carrier body via any suitable fastener(s) 80 (e.g., a zipper or one or more buttons, clips, etc.) to retain the cover 66 in the closed position 84. In some optional aspects, the cover 66 can be retained in an open position 86. For example, the carrier 12 can comprise a fastener 82 (e.g., hook and loop material, a snap, or one or more magnets) that retains the cover 66 in the open position 86. In further optional aspects, the cover 66 can be spring biased toward the open position 86 or locked in the open position via a releasable mechanical catch. Optionally, the cover 66 can comprise a lip 88 that extends below the top opening 68 relative to a vertical axis when the cover 66 is in the closed position 84.

In various aspects, the carrier body 14 can define at least one coupling element that enables different coupling apparatuses to be attached thereto and removed therefrom. The coupling element can complise, for example, a loop through which the strap 30 or the belt 32 can be inserted. In further optional aspects, the coupling element can comprise a carabineer, a clip (e.g., a double side release buckle), and/or one ofa hook or loop fastener that is configured to engage the other of the hook or loop fastener of the coupling apparatus 26.

Conventional electric-signal generator carriers resemble luggage and are meant to be removed from the person during typical daily activities. Unlike such conventional electric-signal generator carriers, the carrier 12 disclosed herein is configured to remain on the person during daily activities such as sitting, standing, walking, exercising, etc. Thus, the length of the electrical cable(s) can be reduced to the length necessary to allow unrestricted movement of the subject, without having excess length that can serve as a tripping hazard or get caught on a snag. For example, the length of the longest electrical cable of the electrical cable(s) 24 can be no more than 20 cm longer than the distance from the carrier to the farthest electrode array from the carrier as measured along a path against the body of the subject. In embodiments comprising an electrical connection box 40, the length of the electrical cable 24 can be the sum of the lengths of the first electrical cable 42, the (longest) second electrical cable(s), and the distance between the first and second ports 50, 52. The reduced length of the electrical cables as compared to conventional systems can further reduce the weight of the electrical cables. In further aspects, excess cable length can be received within the carrier 12. For example, in some aspects, the excess length of the cables can be received within the first receiving space 16. In further aspects, the excess length of cables can be received within the second receiving space 51. In yet further aspects, the system can comprise at least one retention strap (or band or wire tie) that is configured to secure the excess cable length in a rolled, folded, or bunched configuration. For example, the excess length of cable can be folded against itself one or more times to define a plurality of adjacent cable segments, and the retention strap can be wrapped around the plurality of adjacent cable segments. In some aspects, die retention strap can comprise a fastener (e.g., hook and loop material, a snap fastener, or a clip that retains the retention strap in a looped configuration. In further aspects, the retention strap can comprise a semi-rigid member (e.g., as in a twist tie) that enables the retention strap to stay wrapped around the excess length of cable. The retention strap can optionally be coupled to the carrier 12. In further aspects, the retention strap can be separate from, and not coupled to, the carrier 12.

In some aspects, a kit can comprise a carrier body 14 as disclosed herein and a plurality of coupling apparatuses 26. In some aspects, each of the coupling apparatuses 26 can be selectively attached to, or decoupled from, the carrier body 14. In this way, the subject can select between the different coupling apparatuses depending on the coupling apparatus and desired position of the carrier body.

It is contemplated that the electric-signal generator 20 can comprise a portable power source, such as a battery. A spare battery 74 can be positioned within one pocket 70 of the carrier. The spare battery 74 can be used to replace a depleted battery of the electric-signal generator 20.

Embodiments disclosed herein can enable subjects receiving TTF therapy to be mobile and active during treatment. For example, unlike the conventional luggage-resembling carrier, the carrier 12 does not have to be picked up and held. Thus, the subject does not have to constantly pay attention to the location of the carrier. Additionally, embodiments disclosed herein can be more discrete as compared to the conventional luggage-resembling carrier. For example, the carrier 12 can have a much more subtle profile that draws less attention than the conventional carrier. Moreover, in some optional aspects, the carrier 12 can be worn underneath clothes, and the electrical cables can similarly be run underneath the clothes so that little or none of the system 10 is visible to an onlooker.

### Method of Use

A carrier 12 as described herein can be fixedly coupled to the body of the subject 100 at a first location. For example, a strap can be positioned across at least a portion of the torso of the body of the subject or at least an upper arm of the body of the subject or around a thigh of the body of the subject. In further aspects, a belt can be positioned around the torso of the body of the subject. In further aspects, a sleeve can be positioned around the upper arm or thigh of the subject. The electric-signal generator 20 can be positioned within the receiving space 16 of the carrier body 14 of the carrier 12.

Each transducer array 22 can be securely placed at a respective second location on the body of the subject, wherein the first location is different from the second location.

Optionally, each transducer array of the plurality of transducer arrays can be positioned at the respective second location before the carrier is fixedly coupled to the first location of the body. In some optional aspects, the second location(s) can be determined by a computing device 1001. For example, the computing device 1001 can instruct a subject or clinician as to the proper positioning for each transducer array. The computing device, can receive (e.g., via clinician input) information regarding the region to be treated, such as, for example, a location of a tumor within the body. Based on the position and the anatomy of the patient, the computing device can determine the proper positioning for each transducer array and provide an output indicative of the proper positioning for each transducer array.

In further optional aspects, the computing device 1001 can determine at least one placement location on the body of the subject where the carrier can be fixedly coupled to avoid overlapping any portion of the plurality of transducer arrays. In some aspects, based on placement locations of the transducer arrays, the computing device can provide locations for positioning the carrier body 14 in which the carrier body is spaced from the transducer arrays (e.g., so that the transducer array is not between the carrier body and the subject). In this way, excess heat in one location on the body of the subject can be minimized. Thus, for example, in aspects in which the placement locations of the transducer arrays are on the chest of the subject, the computing device can provide recommendations of the armpit, the upper arms, or the thighs of the body of the subject as locations for placement of the carrier body 14. The computing device can be in communication with a display, and the computing device can cause the display to provide an output indicative of the placement location(s) on the body where the carrier can be fixedly coupled to avoid overlapping any portion of the plurality of transducer arrays. For example, the display can output a list of locations or a graphic. The computing device can further provide an output indicative of which coupling apparatus or coupling apparatuses can be used to fixedly couple die carrier to the body in said placement location(s).

Optionally, the computing device 1001 can user the determined proper positioning for each transducer array in order to determine the at least one placement location on the body of the subject where the carrier can be fixedly coupled to avoid overlapping any portion of the plurality of transducer arrays. In further aspects, the computing device can be configured to receive a user input of the location of each transducer array in order to determine the at least one placement location on the body of the subject where the carrier can be fixedly coupled to avoid overlapping any portion of the plurality of transducer arrays. For example, die computing device can provide a prompt (e.g., on a display) for a user to input the location of each transducer array, and user (e.g., a clinician) can input, via an input device of the computing device, the location of each transducer array.

The transducer arrays 22 can be coupled to the electric-signal transducer 20. For example, in some optional aspects, the second electrical cables (e.g., electrical cables 44a, 44b, 44c) of the respective transducer arrays can be coupled to the electrical connection box, and the electric-signal generator 20 can be coupled to the electrical connection box 40 via the first electrical cable 42, thereby providing electrical communication between the electric-signal generator and the transducer array(s). In various aspects, the electrical connection box 40 can be clipped to the carrier (e.g., to the belt 32) or positioned within the carrier (e.g., within the receiving space 16).

The electric-signal generator 20 can be used to deliver electrical signals to the transducer array(s) 22, and the transducer array(s) can generate TTFs. U.S. Patent No. 7,565,205, granted July 21, 2009, describes further details for generating and using TTFs to treat cancer.

In some aspects, delivery of the electrical signals to the transducer array(s) 22 can be ceased. Optionally, after delivery of electrical signals while a carrier is coupled to the subject with a first coupling apparatus, the carrier can be coupled to the subject with a different coupling apparatus. For example, the coupling apparatus can be decoupled from the body of the subject. The coupling apparatus can initially be coupled to the body of the subject with a first coupling apparatus, and a second coupling apparatus that is different from the first coupling apparatus can also be used to couple the carrier to the body of the subject. Optionally, the first coupling apparatus can be decoupled from the carrier body prior to coupling the second coupling apparatus to the carrier body (such that the first coupling apparatus and the second coupling apparatus sequentially couple to the carrier body). Optionally, using die second coupling apparatus, the carrier can be positioned in a third position that is different from the first position and the second position. In exemplary aspects, the second coupling apparatus can differ from the first coupling apparatus in fastener type, coupling location, and/or size.

### Computing Device

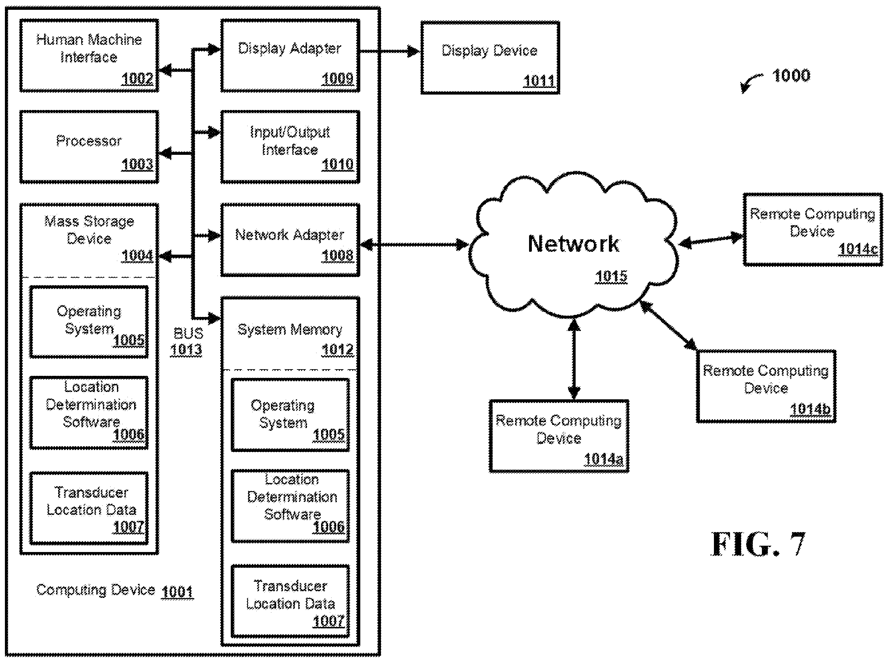

FIG. 7 shows a system 1000 including an exemplary configuration of a computing device 1001 for use with the system 10 disclosed herein. In some aspects, the computing device 1001 can be integral to the system 10. In further aspects, it is contemplated that the computing device 1001 can be a separate computing device, such as, for example, a tablet, smartphone, laptop, or desktop computer can communicate with the system 10 and can enable the subject to interface with the system 10.

The computing device 1001 may comprise one or more processors 1003, a system memory 1012, and a bus 1013 that couples various components of the computing device 1001 including the one or more processors 1003 to the system memory 1012. In the case of multiple processors 1003, the computing device 1001 may utilize parallel computing.

The bus 1013 may comprise one or more of several possible types of bus structures, such as a memory bus, memory controller, a peripheral bus, an accelerated graphics port, and a processor or local bus using any of a variety of bus architectures.

The computing device 1001 may operate on and/or comprise a variety of computer readable media (e.g., non-transitory). Computer readable media may be any available media that is accessible by the computing device 1001 and comprises, non-transitory, volatile and/or non-volatile media, removable and non-removable media. The system memory 1012 has computer readable media in the form of volatile memory, such as random access memory (RAM), and/or non-volatile memory, such as read only memory (ROM). The system memory 1012 may store data such as transducer location data 1007 (i.e., data from signals received by the electrodes) and/or program modules such as operating system 1005 and location determination software 1006 that are accessible to and/or are operated on by the one or more processors 1003.

The computing device 1001 may also comprise other removable/non-removable, volatile/non-volatile computer storage media. The mass storage device 1004 may provide non-volatile storage of computer code, computer readable instructions, data structures, program modules, and other data for the computing device 1001. The mass storage device 1004 may be a hard disk, a removable magnetic disk, a removable optical disk, magnetic cassettes or other magnetic storage devices, flash memory cards, CD-ROM, digital versatile disks (DVD) or other optical storage, random access memories (RAM), read only memories (ROM), electrically erasable programmable read-only memory (EEPROM), and the like.

Any number of program modules may be stored on the mass storage device 1004. An operating system 1005 and location determination software 1006 may be stored on the mass storage device 1004. One or more of the operating system 1005 and location determination software 1006 (or some combination thereof) may comprise program modules and the location determination software 1006. Transducer location data 1007 may also be stored on the mass storage device 1004. Transducer location data 1007 may be stored in any of one or more databases known in the art. The databases may be centralized or distributed across multiple locations within the network 1015.

A user may enter commands and information into the computing device 1001 using an input device (not shown). Such input devices comprise, but are not limited to, a keyboard, touchscreen display, pointing device (e.g., a computer mouse, remote control), a microphone, a joystick, a scanner, tactile input devices such as gloves, and other body coverings, motion sensor, speech recognition, and the like. These and other input devices may be connected to the one or more processors 1003 using a human machine interface 1002 that is coupled to the bus 1013, but may be connected by other interface and bus structures, such as a parallel port, game port, an IEEE 1394 Port (also known as a Firewire port), a serial port, network adapter 1008, and/or a universal serial bus (USB).

A display device 1011 may also be connected to the bus 1013 using an interface, such as a display adapter 1009. It is contemplated that the computing device 1001 may have more than one display adapter 1009 and the computing device 1001 may have more than one display device 1011. A display device 1011 may be a monitor, an LCD (Liquid Crystal Display), light emitting diode (LED) display, television, smart lens, smart glass, and/ or a projector. In addition to the display device 1011, other output peripheral devices may comprise components such as speakers (not shown) and a printer (not shown) which may be connected to the computing device 1001 using Input/Output Interface 1010. Any step and/or result of the methods may be output (or caused to be output) in any form to an output device. Such output may be any form of visual representation, including, but not limited to, textual, graphical, animation, audio, tactile, and the like. The display 1011 and computing device 1001 may be part of one device, or separate devices.

The computing device 1001 may operate in a networked environment using logical connections to one or more remote computing devices 1014a,b,c. A remote computing device 1014a,b,c may be a personal computer, computing station (e.g., workstation), portable computer (e.g., laptop, mobile phone, tablet device), smart device (e.g., smartphone, smart watch, activity tracker, smart apparel, smart accessory), security and/or monitoring device, a server, a router, a network computer, a peer device, edge device or other common network node, and so on. Logical connections between the computing device 1001 and a remote computing device 1014a,b,c may be made using a network 1015, such as a local area network (LAN) and/or a general wide area network (WAN) , or a Cloud-based network. Such network connections may be through a network adapter 1008. A network adapter 1008 may be implemented in both wired and wireless environments. Such networking environments arc conventional and commonplace in dwellings, offices, enterprise-wide computer networks, intranets, and the Internet. It is contemplated that the remote computing devices 1014a,b,c can optionally have some or all of the components disclosed as being part of computing device 1001. In various further aspects, it is contemplated that some or all aspects of data processing described herein can be performed via cloud computing on one or more servers or other remote computing devices. Accordingly, at least a portion of the system 1000 can be configured with internet connectivity.

Although the foregoing invention has been described in some detail by way of illustration and example for purposes of clarity of understanding, certain changes and modifications may be practiced within the scope of the appended claims.

## Claims

1. A system for applying tumor-treating fields to a portion of a body of a subject, the system comprising:
a carrier (12) comprising a carrier body (14) that defines a receiving space (16), and a coupling apparatus (26) that is configured to fixedly couple the carrier body (14) to a first location on the body of the subject;
an electric-signal generator (20) at least partially received within the receiving space (16) of the carrier body (14); and
at least one transducer array (22) in electrical communication with the electric-signal generator (20);
**characterized in that** the first location is one of a thigh, a chest, an upper arm or an armpit of the body of the subject, and each transducer array (22) is configured for secure placement at a respective second location on the body of the subject, different than the first location.

2. The system of claim 1, wherein the coupling apparatus (26) comprises a strap (32) configured for positioning across at least a portion of a torso of the body of the subject or around the upper arm of the body of the subject.

3. The system of claim 1, wherein the coupling apparatus (26) comprises a belt (30).

4. The system of claim 1, wherein the coupling apparatus (26) comprises a sleeve (34) configured for positioning around the upper arm of the body of the subject or around the thigh of the body of the subject.

5. The system of claim 1, wherein the coupling apparatus (26) comprises a belt (30) and a strap (32) coupled to and extending between the carrier body (14) and the belt (30).

6. The system of claim 1, further comprising:
an electrical cable (24) providing electrical communication between the electric-signal generator (20) and the at least one transducer array (22).

7. The system of claim 1, further comprising:
an electrical connection box (40);
a first electrical cable (42) providing electrical communication between the electric-signal generator (20) and the electrical connection box (40); and
at least a second electrical cable (44a-c) providing electrical communication between the electrical connection box (40) and the at least one transducer array (22).

8. The system of claim 7, wherein the coupling apparatus (26) comprises a belt (30), a strap (32) coupled to and extending between the carrier body (14) and the belt (30), and a clip (53) that couples the electrical connection box (40) to the belt (30).

9. The system of claim 8, wherein the electrical connection box (40) is at least partially received within the receiving space (16) of the carrier body (14).

10. The system of claim 7, wherein the carrier (12) further comprises a second receiving space (51) which at least partially receives the electrical connection box (40).

11. The system of claim 7, wherein the electrical connection box (40) has opposing first and second sides (46, 48), with the first side (46) defining first and second electrical ports (50, 52), and the first electrical cable (42) being coupled to the first electrical port (50) and the second electrical cable (44a-c) being coupled to the second electrical port (52).

12. The system of claim 3, wherein the belt (30) is configured for positioning around a portion of a torso of the body of the subject.

13. The system of claim 1, wherein the carrier body (14) has inner and outer surfaces (60, 62), the inner surface (60) defining the receiving space (16) of the carrier body (14), and the carrier body (14) further defines an opening (64) extending between the inner and outer surfaces (60, 62), the opening (64) being in communication with the receiving space (16) and configured to permit dissipation of heat from the electric-signal generator (20).

14. The system of claim 1, wherein the second location is on a head of the body of the subj ect.

15. A kit comprising a system as in any one of claims 1 to 14, wherein the carrier (12) further comprises at least a second coupling apparatus (26) that is configured for selective removable coupling to the carrier body (14), with each of the first and second coupling apparatuses (26) being configured to permit coupling of the carrier body (14) to a different location of the body of the subject.

## Patentansprüche

1. System zum Anlegen von Tumorbehandlungsfeldern an einen Abschnitt eines Körpers eines Subjekts, wobei das System umfasst:
einen Träger (12), umfassend einen Trägerkörper (14), der einen Aufnahmeraum (16) definiert, und eine Kupplungseinrichtung (26), die konfiguriert ist, um den Trägerkörper (14) an einer ersten Stelle an dem Körper des Subjekts fest zu koppeln;
einen elektrischen Signalgenerator (20), der mindestens teilweise in dem Aufnahmeraum (16) des Trägerkörpers (14) aufgenommen ist; und
mindestens eine Wandleranordnung (22) in elektrischer Kommunikation mit dem elektrischen Signalgenerator (20);
**dadurch gekennzeichnet, dass** die erste Stelle eine von einem Oberschenkel, einer Brust, einem Oberarm oder einer Achselhöhle des Körpers des Subjekts ist, und jede Wandleranordnung (22) zur sicheren Platzierung an einer jeweiligen zweiten Stelle auf dem Körper des Subjekts, die sich von der ersten Stelle unterscheidet, konfiguriert ist.

2. System nach Anspruch 1, wobei die Kopplungseinrichtung (26) einen Riemen (32) umfasst, der zum Positionieren über mindestens einen Abschnitt eines Oberkörpers des Körpers des Subjekts oder um den Oberarm des Körpers des Subjekts konfiguriert ist.

3. System nach Anspruch 1, wobei die Kopplungseinrichtung (26) einen Gurt (30) umfasst.

4. System nach Anspruch 1, wobei die Kopplungseinrichtung (26) eine Hülse (34) umfasst, die zum Positionieren um den Oberarm des Körpers des Subjekts oder um den Oberschenkel des Körpers des Subjekts konfiguriert ist.

5. System nach Anspruch 1, wobei die Kopplungseinrichtung (26) einen Gurt (30) und einen Riemen (32), der an den Trägerkörper (14) und den Gurt (30) gekoppelt ist und sich zwischen diesen erstreckt, umfasst.

6. System nach Anspruch 1, weiter umfassend:
ein elektrisches Kabel (24), das elektrische Kommunikation zwischen dem elektrischen Signalgenerator (20) und der mindestens einen Wandleranordnung (22) bereitstellt.

7. System nach Anspruch 1, weiter umfassend:
einen elektrischen Anschlusskasten (40);
ein erstes elektrisches Kabel (42), das elektrische Kommunikation zwischen dem elektrischen Signalgenerator (20) und dem elektrischen Anschlusskasten (40) bereitstellt; und
mindestens ein zweites elektrisches Kabel (44a-c), das elektrische Kommunikation zwischen dem elektrischen Anschlusskasten (40) und der mindestens einen Wandleranordnung (22) bereitstellt.

8. System nach Anspruch 7, wobei die Kopplungseinrichtung (26) einen Gurt (30), einen Riemen (32), der an den Trägerkörper (14) und den Gurt (30) gekoppelt ist und sich zwischen diesen erstreckt, und einen Clip (53) umfasst, der den elektrischen Anschlusskasten (40) an den Gurt (30) koppelt.

9. System nach Anspruch 8, wobei der elektrische Anschlusskasten (40) mindestens teilweise in dem Aufnahmeraum (16) des Trägerkörpers (14) aufgenommen ist.

10. System nach Anspruch 7, wobei der Träger (12) weiter einen zweiten Aufnahmeraum (51) umfasst, der mindestens teilweise den elektrischen Anschlusskasten (40) aufnimmt.

11. System nach Anspruch 7, wobei der elektrische Anschlusskasten (40) gegenüberliegende erste und zweite Seiten (46, 48) aufweist, wobei die erste Seite (46) einen ersten und einen zweiten elektrischen Anschluss (50, 52) definiert und das erste elektrische Kabel (42) an den ersten elektrischen Anschluss (50) gekoppelt ist und das zweite elektrische Kabel (44a-c) an den zweiten elektrischen Anschluss (52) gekoppelt ist.

12. System nach Anspruch 3, wobei der Gurt (30) zum Positionieren um einen Abschnitt eines Oberkörpers des Körpers des Subjekts konfiguriert ist.

13. System nach Anspruch 1, wobei der Trägerkörper (14) eine innere und eine äußere Oberfläche (60, 62) aufweist, wobei die innere Oberfläche (60) den Aufnahmeraum (16) des Trägerkörpers (14) definiert und der Trägerkörper (14) weiter eine Öffnung (64) definiert, die sich zwischen der inneren und der äußeren Oberfläche (60, 62) erstreckt, wobei die Öffnung (64) in Kommunikation mit dem Aufnahmeraum (16) steht und konfiguriert ist, um die Ableitung von Wärme aus dem elektrischen Signalgenerator (20) zu ermöglichen.

14. System nach Anspruch 1, wobei die zweite Stelle auf einem Kopf des Körpers des Subjekts ist.

15. Kit, umfassend ein System nach einem der Ansprüche 1 bis 14, wobei der Träger (12) weiter mindestens eine zweite Kopplungseinrichtung (26) umfasst, die zum selektiven entfernbaren Koppeln an den Trägerkörper (14) konfiguriert ist, wobei jede der ersten und der zweiten Kopplungseinrichtung (26) konfiguriert ist, um Koppeln des Trägerkörpers (14) an eine unterschiedliche Stelle des Körpers des Subjekts zu ermöglichen.

## Revendications

1. Système pour appliquer des champs de traitement de tumeur à une partie du corps d'un sujet, le système comprenant :
un support (12) comprenant un corps de support (14) qui définit un espace de réception (16), et un appareil de couplage (26) qui est configuré pour coupler de manière fixe le corps de support (14) à un premier emplacement sur le corps du sujet ;
un générateur de signal électrique (20) au moins partiellement reçu à l'intérieur de l'espace de réception (16) du corps de support (14) ; et
au moins un réseau de transducteurs (22) en communication électrique avec le générateur de signal électrique (20) ;
**caractérisé en ce que** le premier emplacement est l'un parmi une cuisse, une poitrine, un bras ou une aisselle du corps du sujet, et chaque réseau de transducteurs (22) est configuré pour un placement sécurisé à un second emplacement respectif sur le corps du sujet, différent du premier emplacement.

2. Système selon la revendication 1, dans lequel l'appareil de couplage (26) comprend une sangle (32) configurée pour être positionnée sur au moins une partie du torse du corps du sujet ou autour du bras du corps du sujet.

3. Système selon la revendication 1, dans lequel l'appareil de couplage (26) comprend une ceinture (30).

4. Système selon la revendication 1, dans lequel l'appareil de couplage (26) comprend un manchon (34) configuré pour être positionné autour du bras du corps du sujet ou autour de la cuisse du corps du sujet.

5. Système selon la revendication 1, dans lequel l'appareil de couplage (26) comprend une ceinture (30) et une sangle (32) couplée au corps de support (14) et à la ceinture (30) et s'étendant entre ceux-ci.

6. Système selon la revendication 1, comprenant en outre :
un câble électrique (24) assurant une communication électrique entre le générateur de signal électrique (20) et le au moins un réseau de transducteurs (22).

7. Système selon la revendication 1, comprenant en outre :
un boîtier de connexion électrique (40) ;
un premier câble électrique (42) assurant une communication électrique entre le générateur de signal électrique (20) et le boîtier de connexion électrique (40) ; et
au moins un second câble électrique (44a-c) assurant une communication électrique entre le boîtier de connexion électrique (40) et le au moins un réseau de transducteurs (22).

8. Système selon la revendication 7, dans lequel l'appareil de couplage (26) comprend une ceinture (30), une sangle (32) couplée au corps de support (14) et à la ceinture (30) et s'étendant entre ceux-ci, et une attache (53) qui couple le boîtier de connexion électrique (40) à la ceinture (30).

9. Système selon la revendication 8, dans lequel le boîtier de connexion électrique (40) est au moins partiellement reçu à l'intérieur de l'espace de réception (16) du corps de support (14).

10. Système selon la revendication 7, dans lequel le support (12) comprend en outre un second espace de réception (51) qui reçoit au moins partiellement le boîtier de connexion électrique (40).

11. Système selon la revendication 7, dans lequel le boîtier de connexion électrique (40) présente des premier et second côtés opposés (46, 48), le premier côté (46) définissant des premier et second ports électriques (50, 52), et le premier câble électrique (42) étant couplé au premier port électrique (50) et le second câble électrique (44a-c) étant couplé au second port électrique (52).

12. Système selon la revendication 3, dans lequel la ceinture (30) est configurée pour être positionnée autour d'une partie du torse du corps du sujet.

13. Système selon la revendication 1, dans lequel le corps de support (14) présente des surfaces intérieure et extérieure (60, 62), la surface intérieure (60) définissant l'espace de réception (16) du corps de support (14), et le corps de support (14) définit en outre une ouverture (64) s'étendant entre les surfaces intérieure et extérieure (60, 62), l'ouverture (64) étant en communication avec l'espace de réception (16) et configurée pour permettre la dissipation de la chaleur du générateur de signal électrique (20).

14. Système selon la revendication 1, dans lequel le second emplacement est sur la tête du corps du sujet.

15. Kit comprenant un système selon l'une quelconque des revendications 1 à 14, dans lequel le support (12) comprend en outre au moins un second appareil de couplage (26) qui est configuré pour un couplage amovible sélectif au corps de support (14), chacun des premier et second appareils de couplage (26) étant configuré pour permettre le couplage du corps de support (14) à un emplacement différent du corps du sujet.
